# EUROPEAN PATENT SPECIFICATION

(11) **EP 4 558 202 B1**
(45) Date of publication and mention of the grant of the patent: **17.12.2025**
(21) Application number: 23754833.4
(22) Date of filing: 24.07.2023
(51) Int. Cl.: A61M 15/00, G06M 1/04, G06M 1/08

(54) **DOSE COUNTER**
DOSISZÄHLER
COMPTEUR DE DOSES

(30) Priority: 08.08.2022 GB 202211525
(43) Date of publication of application: 28.05.2025
(73) Proprietor: Merxin Ltd, King's Lynn, Norfolk PE30 5BY (GB)
(72) Inventor: STUART, Adam, King's Lynn PE32 1BS (GB)
(74) Representative: Basck Limited
(86) International application number: PCT/IB2023/057497
(87) International publication number: WO 2024/033729

(56) References cited:
- US-A1- 2012 241 527
- US-A1- 2015 250 959
- US-A1- 2017 128 680
- US-A1- 2019 201 641
- US-A1- 2020 376 209

## Description

### TECHNICAL FIELD

This invention relates to dose counting mechanisms. In particular, though not exclusively, this invention relates to a dose counter for registering a count of doses dispensed from an inhaler and to an inhaler comprising the dose counter.

### BACKGROUND

Patients who use inhalers, such as soft mist inhalers (SMIs), pressurised metered dose inhalers (pMDIs) and dry powder inhalers (DPIs), need to monitor their inhaler usage. Furthermore, regulators of medicines have begun to require that some method of dose indication is included in the inhaler. Dose counters, which provide a precise count of the number of doses remaining, and dose indicators, which provide an indication of the proportion of doses remaining, are well known.

There have been numerous proposals for dose counters to be used with inhalers (whether the inhaler is, for example, a dry powder inhaler, a portable nebuliser, or pressurised metered dose inhaler, or some other type). However, despite progress, there is still an important need for dose counters which are both economic and reliable.

In most dose counters and indicators, the display is indexed each time the inhaler is used. Many dose counters and/or dose indicators are complex, requiring several small mechanical parts. This can increase cost, lead to difficulties in assembly, and often require tight dimensional manufacturing tolerances. Relevant prior art documents are for instance: US 2019/201641 A1, US 2017/128680 A1, US 2012/241527 A1 and US 2015/250959 A1.

Hence, there remains a need for improved dose counters that can overcome the aforementioned drawbacks. It is an object of the invention to address at least one of the above problems, or another problem associated with the prior art.

### SUMMARY OF THE INVENTION

A first aspect of the invention provides a dose counter for registering a count of doses dispensed from an inhaler. The dose counter comprises a counter ring comprising one or more circumferential drive features. The dose counter also comprises a worm comprising a shaft and a gear extending in a helical path about the axis of the shaft for driving the counter ring. The gear comprises one or more recesses distributed along the helical path of the gear. The dose counter further comprises a cylindrical body comprising an engagement feature for driving the gear, the engagement feature extending in a helical or spiral path from the cylindrical body to engage the gear such that relative rotational movement between the cylindrical body and the worm rotates the worm about its shaft.

In this way, the dose counter may advantageously utilise rotational motion of the cylindrical body to operate and drive the counter ring. Significantly, this may allow the remaining number of inhaler doses to be measured with a reduced number of components. This in turn may advantageously simplify manufacture of the dose counter. Moreover, such a dose counter may suitably be utilised wherever a priming operation of the inhaler results in some rotary motion, and may therefore be applicable to many types of inhalers.

In some embodiments, the dose counter may comprise a transfer mechanism for translating a linear or reciprocating motion of the inhaler to a rotary motion of the cylindrical body. This may advantageously allow the cylindrical body to be rotated where a priming operation of the inhaler is linear or reciprocating.

The term "dose counter" as defined herein is taken to include both mechanisms that use a numeric count to indicate the number of doses remaining, as well as dose indicating mechanisms that do not enumerate the number of actuations. Such dose indicating mechanisms may, for example, use colour coding or other means to provide an indication of the proportion of doses remaining or to indicate that a device is ending its useful life.

Suitably, the gear may comprise two, or three, or four, or five, or six, or seven, or eight, or nine, or even ten recesses distributed along the helical path of the gear (i.e. the helical path of gear the about the axis of the shaft of the worm). In some embodiments, the recesses may be uniformly distributed along the helical path of the gear.

Suitably the gear may comprise a plurality of teeth distributed along the helical path of the gear. Thus, the one or more recesses may be defined by a gap or gaps between the plurality of teeth.

In some embodiments, the driving of the counter ring by the worm may be constrained to be in one direction. For example, the driving of the counter ring by the worm may be constrained to be in one direction by a ratchet. Suitably, the ratchet may comprise a pawl configured to engage the counter ring, the worm and/or the cylindrical body to prevent back rotation of the counter ring.

In some embodiments, the engagement feature may comprise a ribbed worm tooth.

In some embodiments, the engagement feature may have, generally orthogonal to its helical or spiral path, a cross section comprising an engagement shape. The engagement shape may suitably be configured to fit into each recess of the gear of the worm. For example, the orientation of the engagement shape may change along the path of the engagement feature, thereby maintaining fit of the engagement shape into at least one of the one or more recesses of the gear as the worm rotates about its shaft.

In some embodiments, the orientation of the engagement shape may change along the path of the engagement feature by unidirectional rotation.

Suitably, the engagement shape may be configured to fit snugly into each recess.

In some embodiments, the engagement shape may be generally square-shaped. For example, the engagement feature may comprise a ribbed worm tooth having a generally square-shaped cross section. In such embodiments, the recess may define a generally right angled corner (i.e. have a cross section along the helical path of the gear in the form of a right angled corner). For example, the recess may be generally L -shaped in cross section.

In some embodiments, the engagement shape may be generally triangular. For example, the engagement feature may comprise a ribbed worm tooth having a generally triangular cross section. In such embodiments, the recess may be generally acute angled (i.e. have a cross section along the helical path of the gear in the form of a peak of a triangle). For example, the recess may be generally < -shaped in cross section.

In some embodiments the engagement shape may have a generally involute profile.

In some embodiments, the engagement feature may be configured to engage the gear simultaneously at at least two different positions along the helical or spiral path of the engagement feature.

In some embodiments, the cylindrical body may comprise a plurality of engagement features. For example, the cylindrical body may comprise two, or three, or four, or five, or six, or seven, or eight, or nine, or even ten engagement features. In such embodiments, the plurality of engagement features may extend in rotationally parallel helical or spiral paths.

In some embodiments, the cylindrical body may comprise two engagement features configured to engage the gear simultaneously. For example, the two engagement features may be configured to engage the gear at at least one position along the helical or spiral path of each of the two engagement features.

In some embodiments, the helical path of the gear may extend less than a complete turn about the axis of the shaft of the worm. In some embodiments, the helical path of the gear may extend a complete turn about the axis of the shaft of the worm.

In some embodiments, the helical path of the gear may extend a complete turn or more about the axis of the shaft of the worm. This may advantageously ensure engagement at all times throughout the motion.

In some embodiments, the dose counter may comprise a housing. Suitably, the worm may be mounted in the housing.

In some embodiments, the dose counter may comprise a locking arm coupled to the housing. Suitably, the locking arm may be engageable with the engagement feature of the cylindrical body upon registration of a final dose. This may advantageously prevent further movement (e.g. rotation) of the cylindrical body, for example, once a predetermined count of doses have been dispensed from the inhaler.

In some embodiments a feature of the counter ring may be arranged to release a biasing component or element configured to engage with the cylindrical body to prevent further movement (e.g. rotation) of the cylindrical body relative to the counter ring upon registration of a final dose. This may advantageously prevent further movement of the cylindrical body once a predetermined count of doses have been dispensed from the inhaler. Suitably, the feature on the counter ring may comprise a missing tooth, a filled in tooth or a break or boss protruding from the counter ring. The biasing component or element may comprise a spring or a stamped metal component. The biasing component or element may be configured to engage with the or an engagement feature or with another feature of the cylindrical body.

In some embodiments, the dose counter may comprise a sprung metal component. In such embodiments, a portion of the circumference of the counter ring may comprise a gap for receiving the sprung metal component. Suitably, the sprung metal component may be anchored to part of an inhaler comprising the dose counter, for example, such as a housing or mouthpiece of the inhaler. In this way, a leading edge of sprung metal component may be arranged to move into the gap and into the path of the engagement feature. This may prevent further movement (e.g. rotation) of the cylindrical body, for example relative to the housing or mouthpiece, upon registration of a final dose. This may advantageously prevent further movement of the cylindrical body once a predetermined count of doses have been dispensed from the inhaler.

In some embodiments, the dose counter may comprise a spring and rigid locking component. The spring and rigid locking component may suitably be located in a guiding feature. The guiding feature may be fixed to part of an inhaler comprising the dose counter, for example, such as a housing or mouthpiece of the inhaler. The spring may be configured to be released by the counter ring to move into the path of the engagement feature. This may prevent further movement (e.g. rotation) of the cylindrical body, for example relative to the housing or mouthpiece, upon registration of a final dose. This may advantageously prevent further movement of the cylindrical body once a predetermined count of doses have been dispensed from the inhaler. The rigid locking component may be arranged to prevent the spring from disengaging once it has engaged with the cylindrical body.

In some embodiments, the dose counter may comprise a locking clip. The locking clip may suitably be attached to the counter ring. The locking clip may be arranged to be moveable by the worm to a position in which it is engaged with the engagement feature to prevent further movement of the cylindrical body. For example, the locking clip may be arranged such that engagement of the locking clip with the worm moves the locking clip from a first (unlocked) position in which it lies generally flush with the counter ring (i.e. in generally the same plane as the counter ring), such that it does not block the path of the engagement feature, to a second (locked) position in which it is angled with respect to the counter ring, such that it blocks the path of the engagement feature.

In this way, once the locking clip engages with the worm, the worm may remain fixed in place with respect to the counter ring, thereby locking the cylindrical body to the counter ring and preventing further movement (e.g. rotation) of the cylindrical body relative to the counter ring. Suitably, the worm may be mounted on a fixed part of housing or mouthpiece. Thus, locking the cylindrical body to the counter ring may prevent further movement (e.g. rotation) of the cylindrical body relative to the housing or mouthpiece, upon registration of a final dose.

In some embodiments, the cylindrical body may be part of a secondary housing of the dose counter.

In some embodiments, the cylindrical body may be hollow.

In some embodiments, the engagement feature may suitably extend from an exterior surface of the cylindrical body.

In some embodiments, the counter ring and the cylindrical body may share a common axis. For example, the counter ring and the cylindrical body may be rotatable about a common axis.

In some embodiments, the counter ring circumferential drive feature may comprise regularly spaced teeth or ribs.

In some embodiments, the circumferential drive feature may extend part way around the circumference of the counter ring. Suitably, the circumferential drive feature may be interrupted by a discontinuity. For example, where the circumferential drive feature comprises regularly spaced teeth or ribs, the circumferential drive feature may be interrupted by a discontinuity in the regular spacing of the teeth or ribs. Suitably, the discontinuity may be in the form of a wider gap or a filled in gap.

In some embodiments, the dose counter may comprise in the range 1 to 12 engagement features. For example, the dose counter may comprise, one, or two, or three, or four, or five, or six, or seven, or eight, or nine, or ten, or eleven, or even twelve engagement features.

In some embodiments, the angular advancement of the counter ring relative to the angular movement of the cylindrical body may be in a ratio in the range of from 2:5 to 1:400, or in the range of 2:5 to 1:300, or in the range of 2:5 to 1:200, or in the range of 2:5 to 1:100, or in the range of 2:5 to 1:80, or in the range of 2:5 to 1:60, or in the range of 2:5 to 1:40, or even in the range of 2:5 to 1:20.

A second aspect of the invention provides an inhaler (i.e. inhalation device) comprising a dose counter according to the first aspect of the invention.

In some embodiments, the inhaler may suitably be configured for nebulising pharmaceutical liquids. For example, the inhaler may be a soft mist inhaler (SMI).

In some embodiments, the inhaler may suitably be configured for the delivery of dry powder medicaments. For example, the inhaler may be a dry powder inhaler (DPI).

Throughout the description and claims of this specification, the words "comprise" and "contain" and variations of the words, for example "comprising" and "comprises", mean "including but not limited to", and do not exclude other components, integers or steps. Moreover, the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible.

### BRIEF DESCRIPTION OF THE DRAWINGS

One or more embodiments of the invention will now be described, by way of example only, with reference to the accompanying drawings, in which:

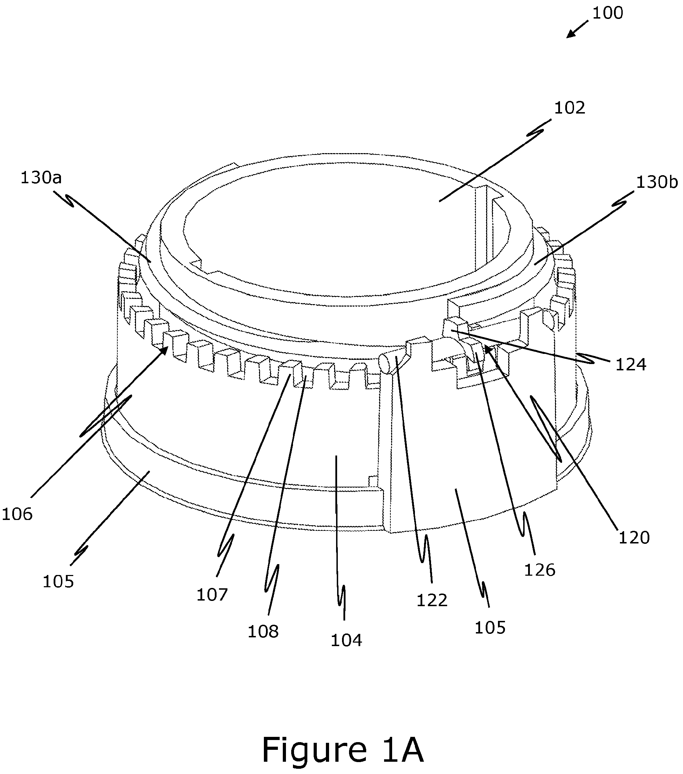

Figure 1A shows a perspective view from above and to one side of a dose counter in accordance with a first embodiment of the invention, the dose counter having a hollow cylindrical body located concentrically within a cylindrical counter ring, the cylindrical body and counter ring arranged concentrically in a housing chassis and rotatable relative to one another, a first end of the cylindrical counter ring comprising a circumferential drive feature having a plurality of equally spaced apart tooth shaped projections that extend from the first end of the counter ring, a worm meshed to the cylindrical body and the counter ring, the worm having a cylindrical shaft. The ends of the cylindrical shaft rotatably mounted in a generally cylindrical slot in the housing.
Figure 1B shows a cross sectional side view of the dose counter of Figure 1A;
Figure 1C shows a top view of the dose counter of Figure 1A;
Figure 1D shows a side view of the dose counter of Figure 1A in a locked position;
Figure 2A shows a perspective view of the cylindrical body of Figures 1A-D;
Figure 2B shows a side view of the cylindrical body of Figures 1A-D;
Figure 3 shows a perspective view of the worm of Figures 1A-D;
Figure 4A shows a side view of the dose counter of Figures 1A-D prior to rotation of the cylindrical body with respect to the counter ring;
Figure 4B shows a side view of the dose counter of Figure 4A after a 90-degree anti-clockwise rotation of the cylindrical body;
Figure 4C shows a side view of the dose counter of Figure 4B after a further 90-degree clockwise rotation of the cylindrical body;
Figure 5A shows a cross sectional view of the dose counter of Figure 1 inside a soft mist inhaler (SMI) device;
Figure 5B shows a partial cross-sectional view of the soft mist inhaler (SMI) device of Figure 5B, which shows the spring cap engaged with the spring cage;
Figure 6A shows a perspective view of a dose counter in accordance with a second embodiment of the invention, the dose counter in this embodiment similar to that of the first embodiment, and having a sprung metal locking component, the sprung metal locking component being shown in a non-locked position;
Figure 6B shows a perspective view of the dose counter of Figure 6A, the sprung metal locking component being shown in a position where the locking component is released by the dose counter, just prior to lockout occurring;
Figure 7 shows a perspective view of a dose counter in accordance with a third embodiment of the invention, the dose counter in this embodiment similar to that of the first and second embodiments, and having a spring biased rigid locking component;
Figure 8A shows a cross sectional view of the dose counter of Figure 6 located inside a soft mist inhaler (SMI) device in which the spring biased rigid locking component is in a non-locked position;
Figure 8B shows a cross sectional view of the dose counter of Figure 6 located inside a soft mist inhaler (SMI) device in which the spring biased rigid locking component is in a locked position;
Figure 9A shows a perspective view of a counter ring and end of life clip in accordance with a fourth embodiment of the invention, the counter ring and end of life clip shown in an unlocked position;
Figure 9B shows a perspective view of the counter ring and end of life clip of Figure 10A in a locked position;

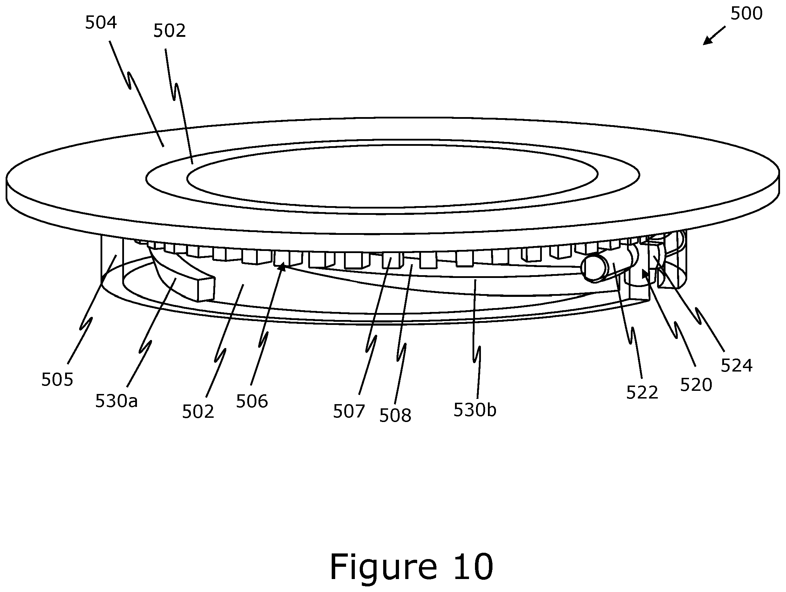

Figure 10 shows a perspective view of a dose counter in accordance with a fifth embodiment of the invention, the dose counter having a disc shaped counter ring having a central cylindrical hole, and a hollow cylindrical body, a first end of the hollow cylindrical body sitting concentrically within the central cylindrical hole of the counter ring, the cylindrical body arranged concentrically in a housing chassis that forms part of a device such as a dry powder inhaler, the cylindrical body and counter ring each rotatable independently with respect to the housing chassis, the outer face of the cylindrical body comprising three engagement features which engage with the teeth of the worm, each of the engagement features comprising a ribbed worm tooth that comprises a square engagement shape;
Figure 11 shows an exploded view of the dose counter of Figure 10;
Figure 12A shows a cross sectional side view of the dose counter of Figure 10 prior to rotation of the cylindrical body with respect to the engagement features;
Figure 12B shows a cross sectional side view of the dose counter of Figure 7A after a 60-degree anti-clockwise rotation of the cylindrical body;
Figure 13A shows a top view of a cylindrical body and worm in accordance with a sixth embodiment of the invention; and
Figure 13B shows a perspective view of the cylindrical body and worm of Figure 13A.

### DETAILED DESCRIPTION

### First Embodiment

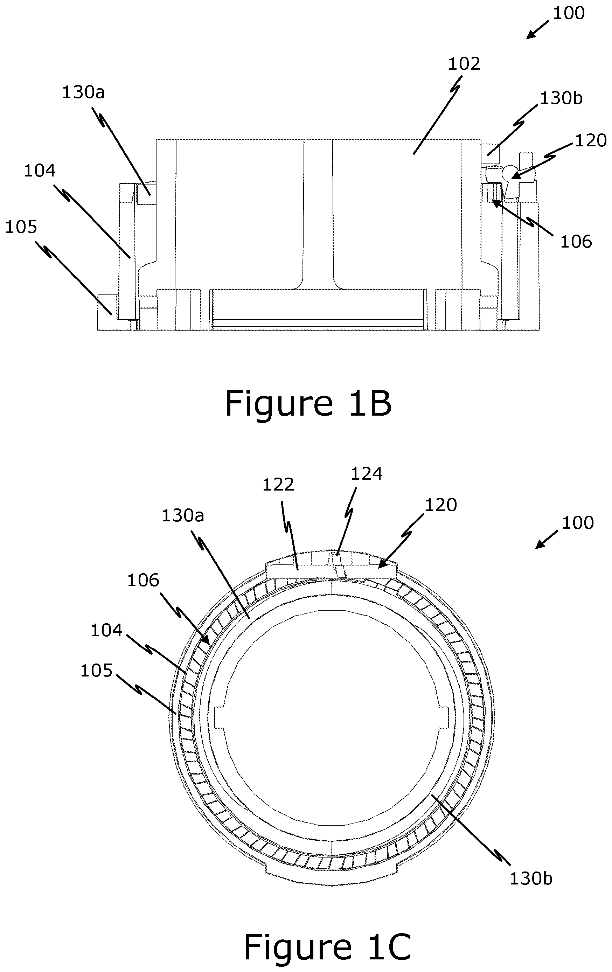

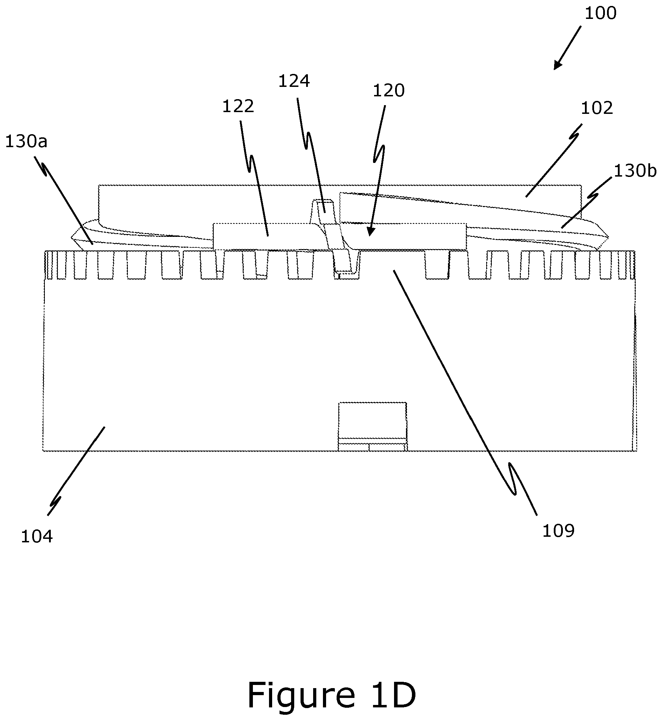

Referring to Figure 1, a dose counter 100 in accordance with a first embodiment of the invention comprises a hollow cylindrical body 102 located concentrically within a cylindrical counter ring 104. The cylindrical body 102 can be rotated with respect to the counter ring 104. The cylindrical body 102 and counter ring 104 are arranged concentrically in a housing chassis 105. The housing chassis 105 may form part of a device (not shown in Figure 1), for example such as a soft mist inhaler (SMI), comprising a supply of liquid medicament and a mouthpiece for delivery of the liquid medicament to a patient. The cylindrical body 102 and counter ring 104 may each rotate independently with respect to the housing chassis 105.

A first end of the cylindrical counter ring 104 comprises a circumferential drive feature 106. In this example, the circumferential drive feature 106 is defined by a plurality of equally spaced apart tooth shaped projections 107 that extend from the first end of the counter ring 104. The plurality of tooth shaped projections 107 define a series of equally spaced apart gaps 108 therebetween. The plurality of tooth shaped projections 107 extend most of the circumference of the first end of the counter ring 104. The counter ring 104 has an outer face printed with numerical indicators. The printed outer face provides an indication of the number of doses of liquid medicament dispensed.

The dose counter 100 also comprises a worm 120 meshed to both the cylindrical body 102 and the counter ring 104. The worm 120 has a cylindrical shaft 122 and a gear 124. The ends of the cylindrical shaft 122 are rotatably mounted in a generally cylindrical slot 150 in the housing 105. The gear 124 meshes into the gaps 108 and engages with the tooth shaped projections 107 of the circumferential drive feature 106. The gear 124 extends in a generally helical path 360° about a mid-point of the axis of the shaft 122 and has four teeth 126 uniformly distributed along the helical path.

The outer face of the cylindrical body 102 comprises a pair of engagement features 130a, 130b which engage with the teeth 126 of the worm 120. **In** this example, each of the engagement features 130a, 130b is a ribbed worm tooth that comprises a square engagement shape (i.e. square shaped in cross section) and extends in a helical path 180° around opposites sides of the outer face of the cylindrical body 102. Each of the engagement features 130a, 130b undergo a 90° twist as they extend around the outer side of the cylindrical body 102. The twist of the engagement features 130a, 130b maintains a snug engagement between two adjacent ones of the teeth 126 of the worm 120 as the cylindrical body 102 is rotated.

Figures 1B and 1C provide a cross sectional side and top views of the dose counter 100 respectively.

Figure 1D provides a side view of the dose counter 100. As shown in Figure 1D, a portion of the circumference of the counter ring 104 provides a lengthened tooth portion 109, which does not have any gaps and defines an end-of-life locking feature. In Figure 1D, the worm 120 is shown abutting the lengthened tooth portion 109, which prevents further anti-clockwise rotation of the cylindrical body 102 with respect to the counter ring 104. In use, this causes the dose counter 100 to become deliberately jammed. When the dose counter 100 is assembled within a soft mist inhaler (SMI) comprising a liquid medicament, this can advantageously prevent the release of further liquid medicament from the inhaler once the inhaler has dispensed a predetermined number of doses of the liquid medicament.

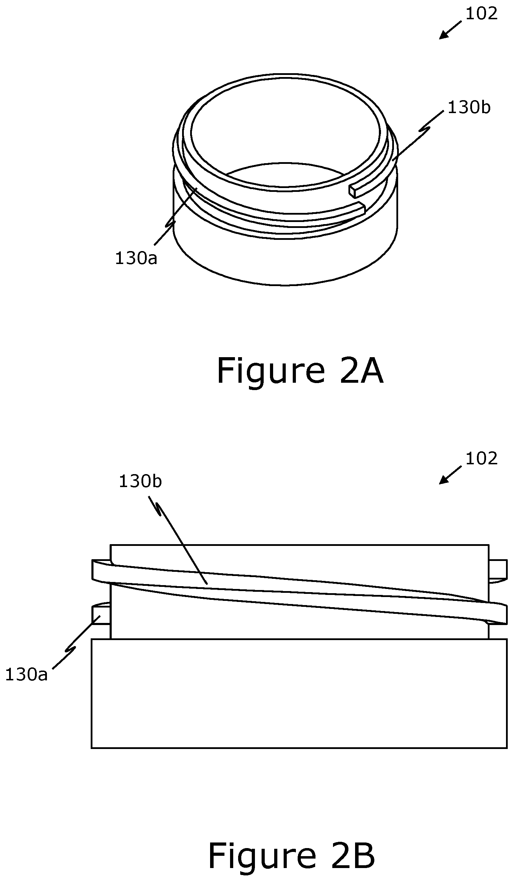

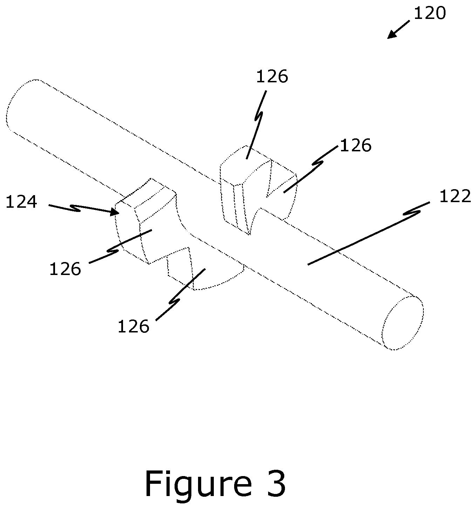

Figures 2A and 2B provide perspective and side views of the cylindrical body 102 respectively, whilst Figure 3 provides a perspective view of the worm 120.

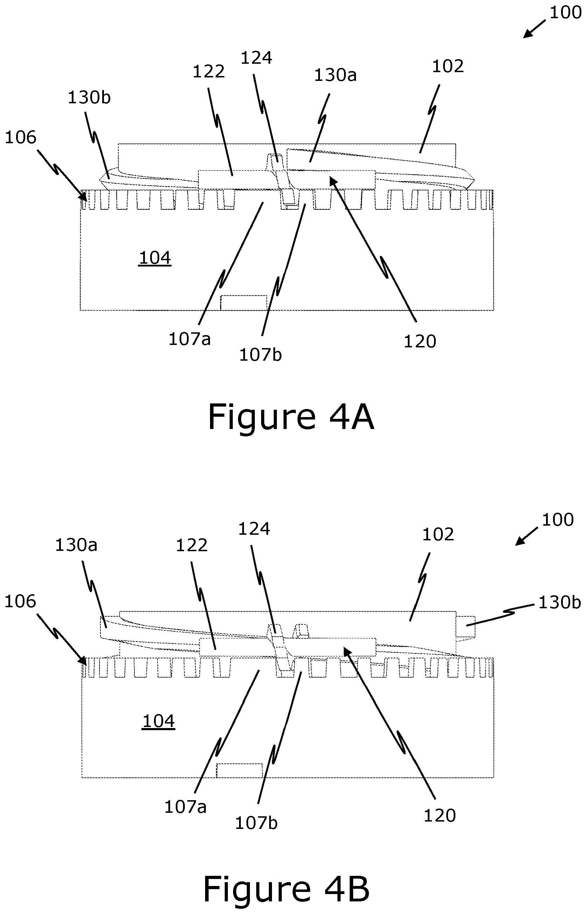

Referring now to Figures 4A-C, in use, the worm 120 starts off engaged between first and second teeth 107a, 107b of the circumferential drive feature 106 (as shown in Figure 4A). In order to use a device (not shown in Figures 4A-C) comprising the dose counter 100, for example such as a soft mist inhaler (SMI), a patient rotates a base portion of the device 180° anti-clockwise to provide a dose of medicament from the device through a mouthpiece of the device.

The base portion of the device is attached to the cylindrical body 102, such that when the base portion is rotated 180° anti-clockwise, the cylindrical body 102 also rotates 180° clockwise with respect to the housing chassis (not shown). As the cylindrical body 102 rotates 180°, the engagement feature 130a of the cylindrical body 102 drives the gear 124 of the worm 120, causing the worm 120 to rotate 90°, i.e. ¼ of a pitch between the teeth 107a, 107b (as shown in Figure 4B).

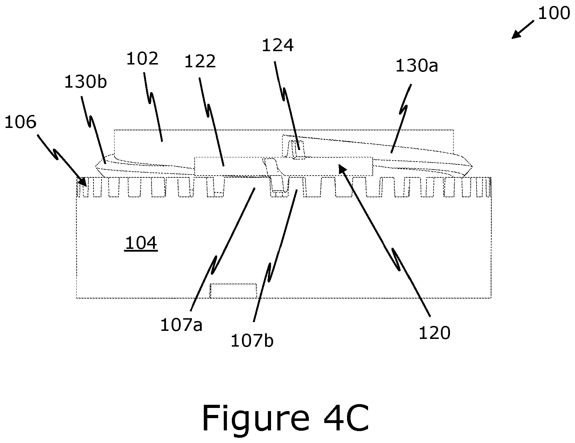

Figure 4C shows the dose counter 100 after a further 90 degrees (total 180 degrees) anti-clockwise rotation of the base portion and hence the cylindrical body 102.

Due to the small increments of rotation, the outer surface of the counter ring 104 is printed with numbers displayed in increments of 5, 10 or 20 to allow the patient to determine the number of doses remaining. It may be feasible to have individual numbers (i.e. 1, 2, 3) printed for the lower geared options, for example such as in the 2:5 ratio - 1:30 ratio range.

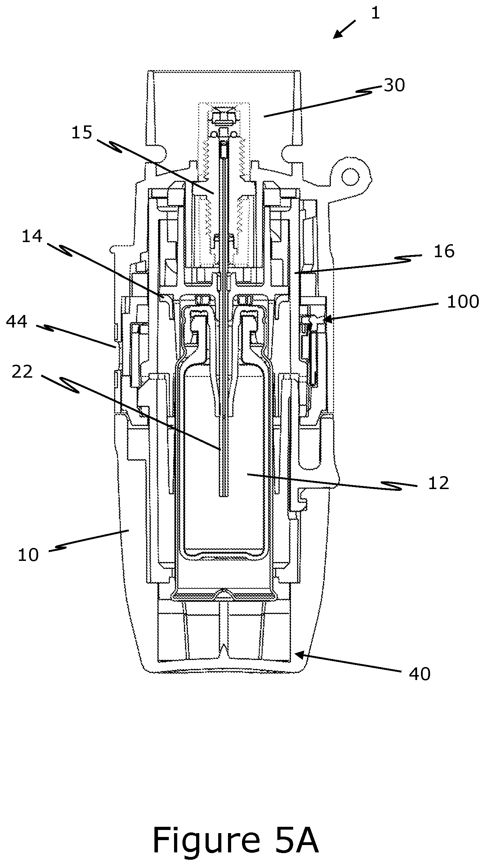

Figure 5A provides a cross sectional view of the dose counter 100 assembled within a soft mist inhaler (SMI) device 1. The device 1 comprises an outer casing 10, a lower half of which houses a cartridge 12 containing a liquid medicament. An upper half of the outer casing 10 comprises a spring cage 16. The spring cage 16 sits directly above the cartridge 12. The dose counter 100 is fixed in position concentrically around the spring cage 16 by the outer casing 10. A spring cap 14 and helical spring (not visible in Figure 5A) are arranged concentrically within the spring cage 16. The spring cap 14 is an annular component which sits over an upper end of the spring cage 16. A conduit 22 fluidly connects the cartridge 12 to a mouthpiece 30 of the device 1. The conduit 22 passes through the centre of the spring cap 14 and helical spring.

The lowermost end of the outer casing 10 is attached to a base portion 40 which is rotatable with respect to the upper half of the outer casing 10. Rotation of the base portion 40 180° clockwise relative to the upper half of the outer casing 10 forces the cartridge 12 to move downwards, thereby compressing the helical spring 17 and drawing up a predetermined volume of liquid medicament from the cartridge 12 through the conduit 22 and out through a micropump 15 located inside the mouthpiece 30. Once the device is actuated, the liquid medicament is expelled from the micropump 15 and device 1, and the helical spring un-compresses and the cartridge 12 returns back to its previous position. The spring cap 14 is rotationally aligned to the spring cage 16 to prevent undesirable torque being transmitted to the device mechanism during actuation of the device 1.

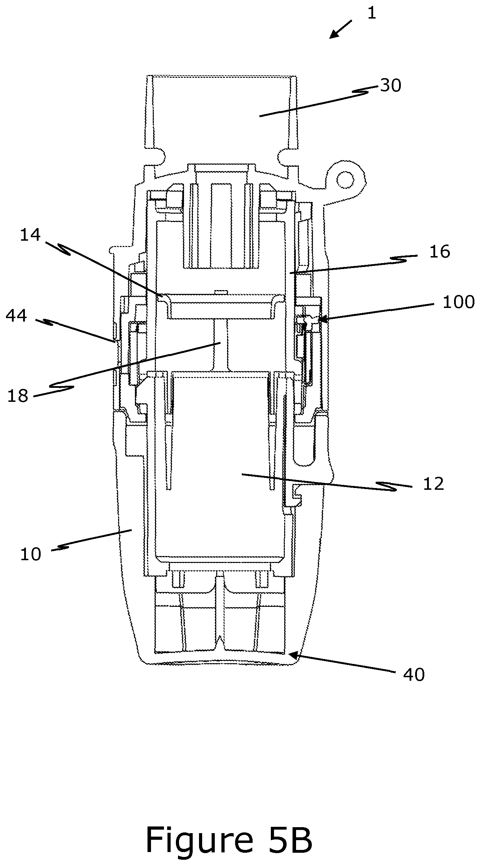

Figure 5B is a partial cross-sectional view of the dose counter 100 assembled within the soft mist inhaler (SMI) device 1, which shows the spring cap 14 engaged with the spring cage 16.

The spring cap 14 has a number of projecting ribs (not visible in Figure 5B) which engage with and run in grooves 18 provided in the spring cage 16.

The base portion 40 is connected to the cylindrical body 102 of the dose counter 100, such that rotation of the base portion 40 180° clockwise causes the cylindrical body 102 to rotate 180° clockwise with respect to the counter ring 104. As the cylindrical body 102 rotates 180°, the engagement feature 130a of the cylindrical body 102 drives the gear 124 of the worm 120, causing the worm 120 to rotate 90°, i.e. ¼ of a pitch between the teeth 107a.

A slot 44 in the outer casing allows numerical indicators printed on the outer face of the counter ring 104 to be read through the outer casing. In this example, the numerical indicators provide an indication of the number of doses of the liquid medicament that have been dispensed from the device 1.

### Second Embodiment

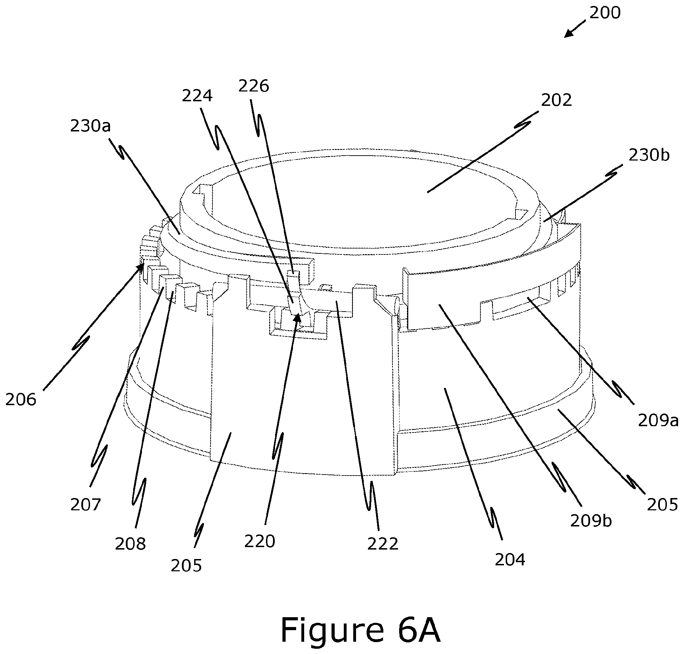

Referring to Figure 6A, a dose counter 200 in accordance with a second embodiment of the invention has generally the same structure as the dose counter 100 of the first embodiment described above, the dose counter 200 having a hollow cylindrical body 202 located concentrically within a cylindrical counter ring 204. The cylindrical body 202 can be rotated with respect to the counter ring 204. The cylindrical body 202 and counter ring 204 are arranged in a housing chassis 205. The housing chassis 205 may form part of a device (not shown in Figure 6A), for example such as a soft mist inhaler (SMI) comprising a supply of liquid medicament and a mouthpiece for delivery of the liquid medicament to a patient.

A first end of the cylindrical counter ring 204 comprises a circumferential drive feature 206. As with the dose counter 100 described above in relation to the first embodiment of the invention, the circumferential drive feature 206 is defined by a plurality of equally spaced apart tooth shaped projections 207 that extend from the first end of the counter ring 204. The plurality of tooth shaped projections 207 define a series of equally spaced apart gaps 208 therebetween. The plurality of tooth shaped projections 207 extend most of the circumference of the first end of the counter ring 204. A portion of the circumference of the counter ring 204 comprises a gap 209a for receiving a sprung metal component 209b. The sprung metal component 209b defines an alternative end-of-life locking feature to the lengthened tooth portion 109 described above in relation to the first embodiment.

The sprung metal component 209 is anchored to part of the device (not shown in Figure 6A), for example such as the mouthpiece. The spung metal component 209b is held out on diameter by the plurality of tooth shaped projections 207 at the start of life (i.e. prior to the device being used) and whilst the dose counter 200 is counting down through life (i.e. during use of the device).

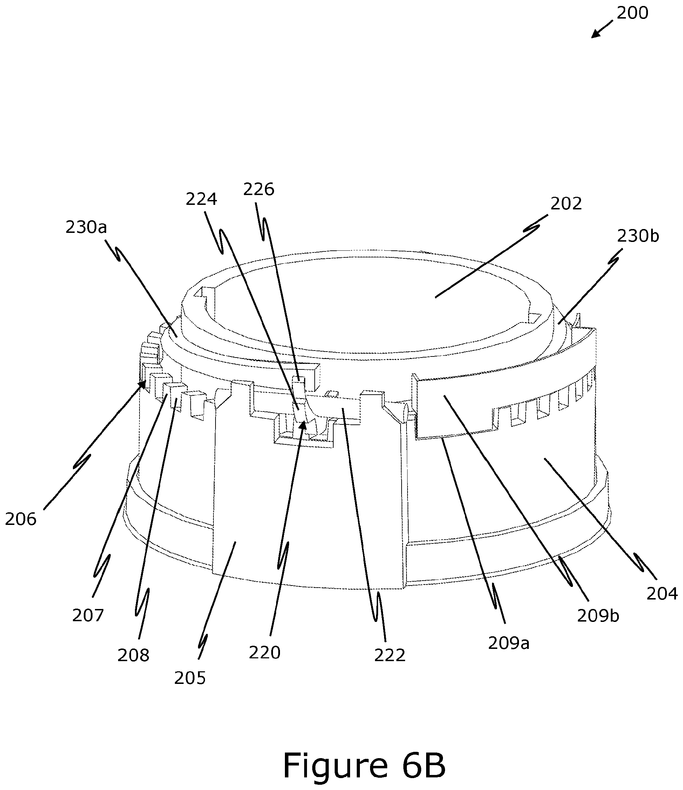

Once the dose counter 200 reaches a display of zero, a leading edge of the sprung metal component 209b moves in on diameter into the gap 209a, thereby blocking the path of further rotation of the cylindrical body 202 relative to the counter ring 204 (as shown in Figure 6B). The sprung metal component 209b engages with a leading face of the helical path of the cylindrical body 202 and prevents the cylindrical body 202 from rotating any further relative to a fixed part of the device (not shown in Figure 6B), for example the mouthpiece. This advantageously prevents the release of further liquid medicament from the device once it has dispensed a predetermined number of doses of the liquid medicament.

### Third Embodiment

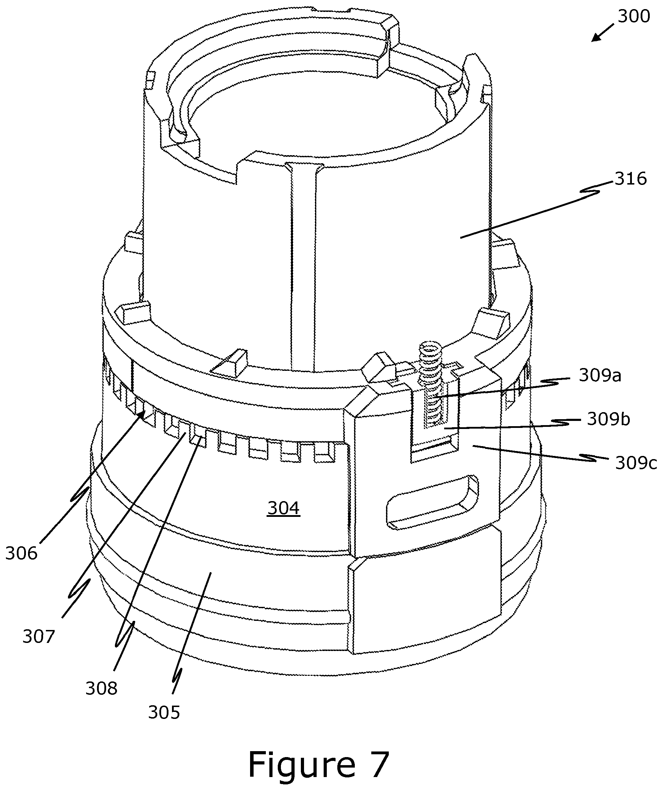

Referring to Figure 7, a dose counter 300 in accordance with a third embodiment of the invention has a spring cage 316 comprising a cylindrical body 302. The cylindrical body 302 is located concentrically within a cylindrical counter ring 304. The cylindrical body 302 can be rotated with respect to the counter ring 304. The cylindrical body 302 and counter ring 304 are arranged concentrically in a housing chassis 305. The housing chassis 305 may form part of a device 51 (not shown in Figure 7), for example such as a soft mist inhaler (SMI) comprising a supply of liquid medicament and a mouthpiece for delivery of the liquid medicament to a patient.

A first end of the cylindrical counter ring 304 comprises circumferential drive feature 306. As with the dose counter 100 described above, the circumferential drive feature 306 is defined by a plurality of equally spaced apart tooth shaped projections 307 that extend from the first end of the counter ring 304. The plurality of tooth shaped projections 307 define a series of equally spaced apart gaps 308 therebetween. The plurality of tooth shaped projections 307 extend most of the circumference of the first end of the counter ring 304. The dose counter 300 comprises a spring 309a and a rigid locking component 309b, which together define an alternative end-of-life locking feature to the lengthened tooth portion 109 described above in relation to the first embodiment.

The spring 309a and rigid locking component 309b are located in a guiding feature 309c fixed to part of the device (not shown in Figure 7), for example such as the mouthpiece. The spring 309a biases the rigid locking component 309b towards the tooth shaped projections 307 of the counter ring 304.The presence of the tooth shaped projections 307 on the counter ring 304 prevents movement of the rigid locking component 309b at start of life (i.e. prior to the device being used) and whilst the counter is counting down through life (i.e. during use of the device).

Once the dose counter 300 reaches a display of zero, there are missing tooth shaped projections 307 on the counter ring 304. The missing tooth shaped projections 307 allow the rigid locking component 309b to be driven by the spring 309a into a locking position where the rigid locking component 309b and block the path of rotation of the cylindrical body 302 relative to a fixed part of the device (not shown in Figure 7). The rigid locking component engages with a leading face of the helical path cylindrical body 302 and prevents the cylindrical body 302 from rotating any further relative to a fixed part of the device (not shown in Figure 7). This advantageously prevents the release of further liquid medicament from the device 51 once it has dispensed a predetermined number of doses of the liquid medicament.

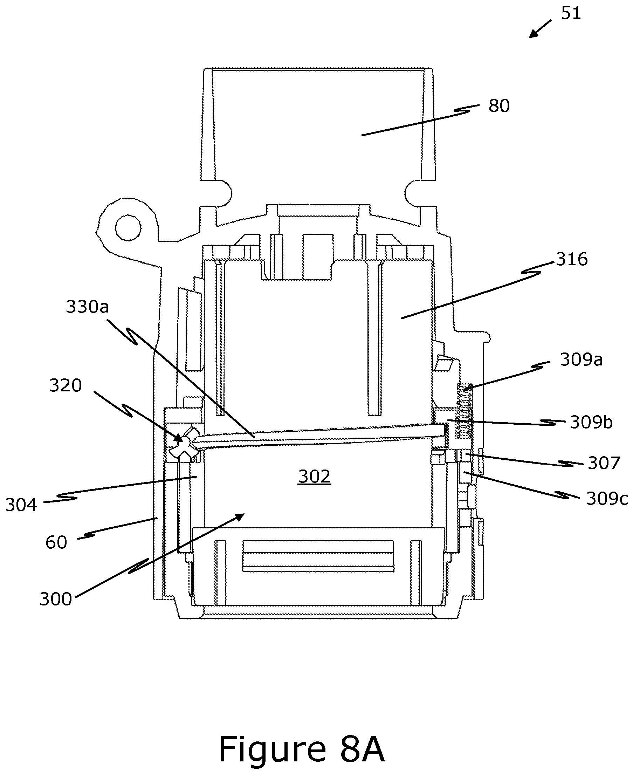

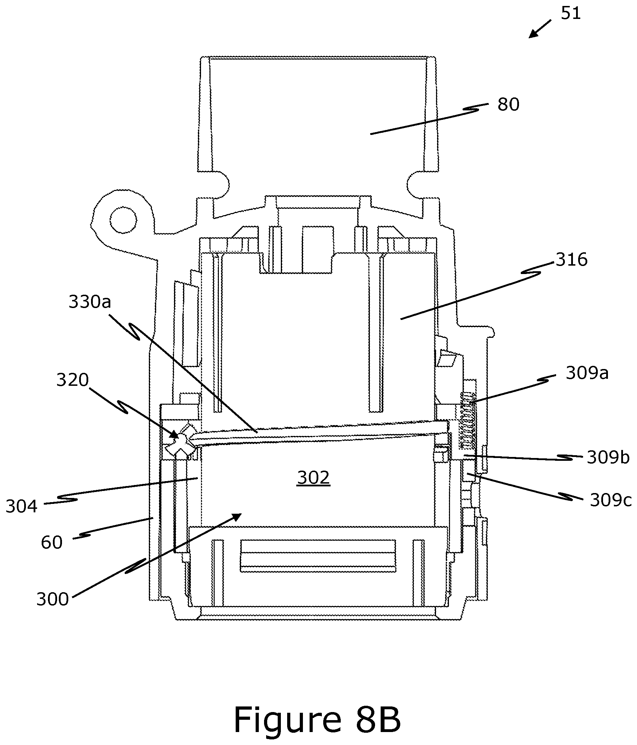

Figures 8A and 8B provide a cross sectional views of the dose counter 300 assembled within a soft mist inhaler (SMI) device 51. The device 51 comprises an outer casing 60, the upper half of which comprises a micropump 15 and nozzle (not shown in Figure 8A and 8B). The dose counter 300 is fixed in position within the device 51 by the outer casing 60. Figure 8A shows the rigid locking component 309b sitting above the tooth shaped projections 307 on the counter ring 304 in a first non-locking position. Figure 8B shows the rigid locking component 309b in a second locking position in which the rigid locking component 309b has been biased downwards by the spring into a gap defined by missing tooth shaped projections 307 on the counter ring 304.

### Fourth Embodiment

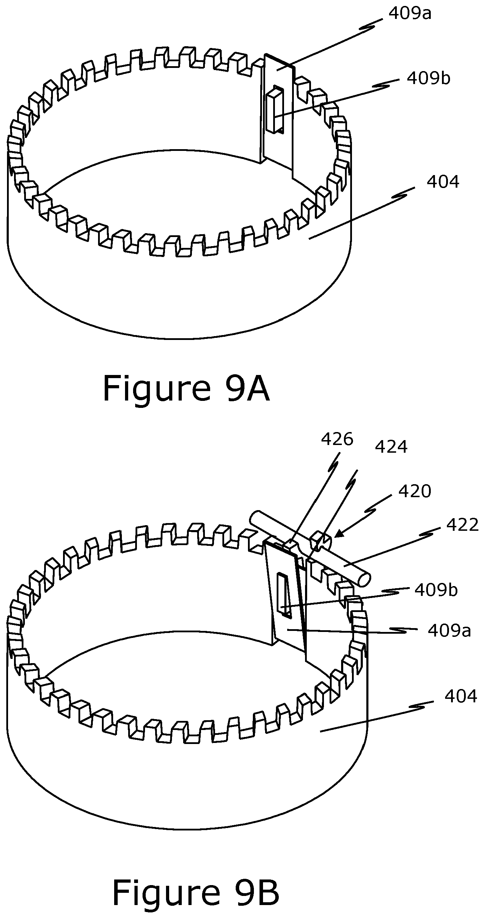

Figures 9A and 9B show a counter ring 404 in accordance with a fourth embodiment of the invention. The counter ring 404 comprises a locking clip 409a attached to the counter ring. The locking clip 409a travels around with the counter ring 404 until it reaches a position where it collides with the path of the worm gear tooth 426. The worm gear tooth 426 flexes one end of the locking clip 409a towards the axis of the cylindrical body 402 (not shown in Figures 10A and 10B) and into the path of the engagement features 430 of the cylindrical body 402, preventing further rotation of the cylindrical body 402 relative to the counter ring 404.

As shown in Figure 9B, the locking clip 409a prevents rotation of the cylindrical body 402 because the locking clip 409a remains engaged with the counter ring 404, which in turn remains engaged with the worm gear 420 and the worm gear 420 is held securely by part of a device in which the counter ring 404 is assembled, for example such as the mouthpiece. The counter ring 404 may include an optional rib 409b to provide improved engagement between the locking clip 409a and the counter ring 404 in its locked position.

### Fifth Embodiment

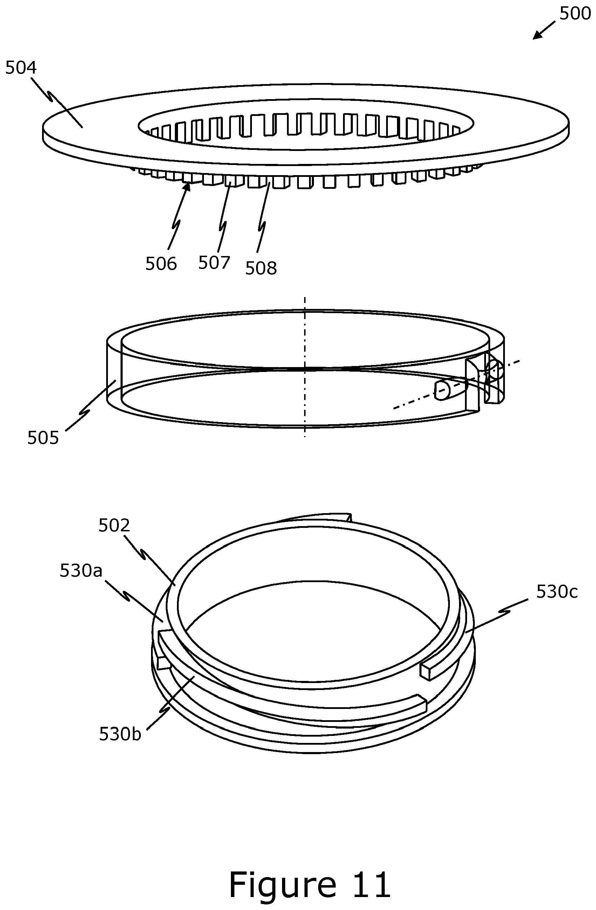

Referring to Figures 10 and 11, a dose counter 500 in accordance with a fifth embodiment of the invention has a disc shaped counter ring 504 having a central cylindrical hole. The dose counter also has a hollow cylindrical body 502, a first end of which sits concentrically within the central cylindrical hole of the counter ring 504. The cylindrical body 502 is arranged concentrically in a housing chassis 505. The housing chassis 505 may form part of a device (not shown in Figures 10 and 11), for example such as a dry powder inhaler (DPI), comprising a supply of medicament powder and a mouthpiece for delivery of the medicament powder to a patient. The cylindrical body 502 and counter ring 504 may each rotate independently with respect to the housing chassis 505.

The counter ring 504 comprises a circumferential drive feature 506 which extends the circumference of the circular hole on a side of the counter ring 504 facing the hollow cylindrical body 502. In this example, the circumferential drive feature 506 is defined by plurality of equally spaced apart tooth shaped projections 407 that project outwardly from the side of the counter ring 504 facing the hollow cylindrical body 502. The plurality of tooth shaped projections 507 define a series of equally spaced apart gaps 408 therebetween. A side of the counter ring 504 is printed with numerical indicators, which provide an indication of the number of doses of medicament powder dispensed.

The dose counter 500 also comprises a worm 520 meshed with both the cylindrical body 502 and the counter ring 504. The worm 520 has a cylindrical shaft 522 and a gear 524. The ends of the cylindrical shaft 522 are rotatably mounted in a cylindrical slot 550 in the housing 505. The gear 524 meshes with the gaps 508 and engages with the tooth shaped projections 507 of the circumferential drive feature 506. The gear 524 extends in a generally helical path 360° about a mid-point of the axis of the shaft 522 and has four teeth 526 uniformly distributed along the helical path.

The outer face of the cylindrical body 502 comprises three engagement features 530a, 530b, 530c which engage with the teeth 526 of the worm 520. In this example, each of the engagement features 530a, 530b, 530c is a ribbed worm tooth that comprises a square engagement shape (i.e. is square shaped in cross section) and extends in a helical path 120° around its own 1/3 segment of the outer face of the cylindrical body 502. As each of the engagement features 530a, 530b, 530c extends around the outer side of the cylindrical body 502, it undergoes a 90° twist. The twist of the engagement features 530a, 530b, 530c helps to maintain a snug engagement between two of the teeth 526 of the worm 520 as the cylindrical body 502 is rotated.

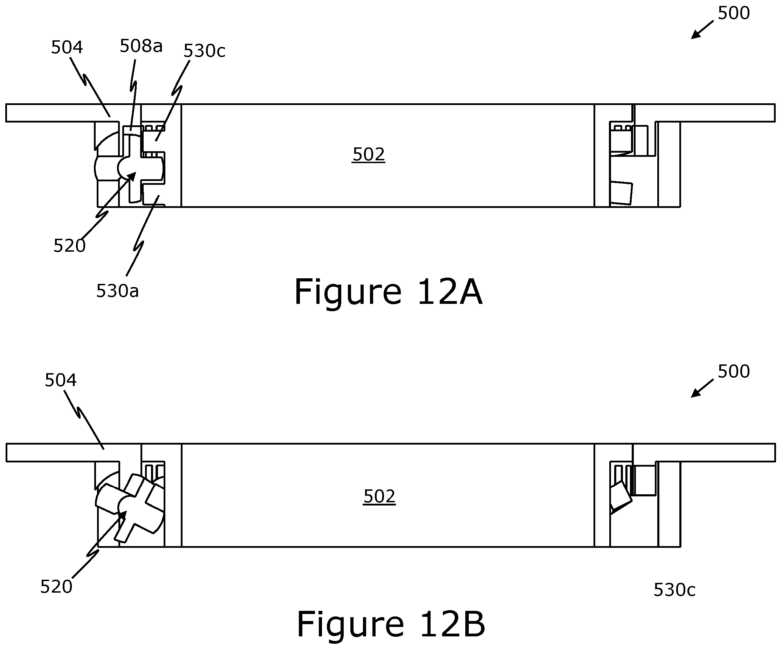

Referring now to Figures 12A and 12B, in use, the worm 520 starts off engaged with a first tooth 507a of the circumferential drive feature 506 (as shown in Figure 12A). In order to use a device (not shown in Figures 12A and 12B) comprising the dose counter 500, for example such as a dry powder inhaler (DPI), opening a cover of the mouthpiece of the device may be arranged to rotate the cylindrical body 502 rotates 120° clockwise with respect to the counter ring 504. As the cylindrical body 502 rotates 120°, the engagement feature 530a of the cylindrical body 502 drives the gear 524 of the worm 520, causing the worm to rotate 90°. Figure 12B shows a point at approximately 60° into the 120° rotation.

### Sixth Embodiment

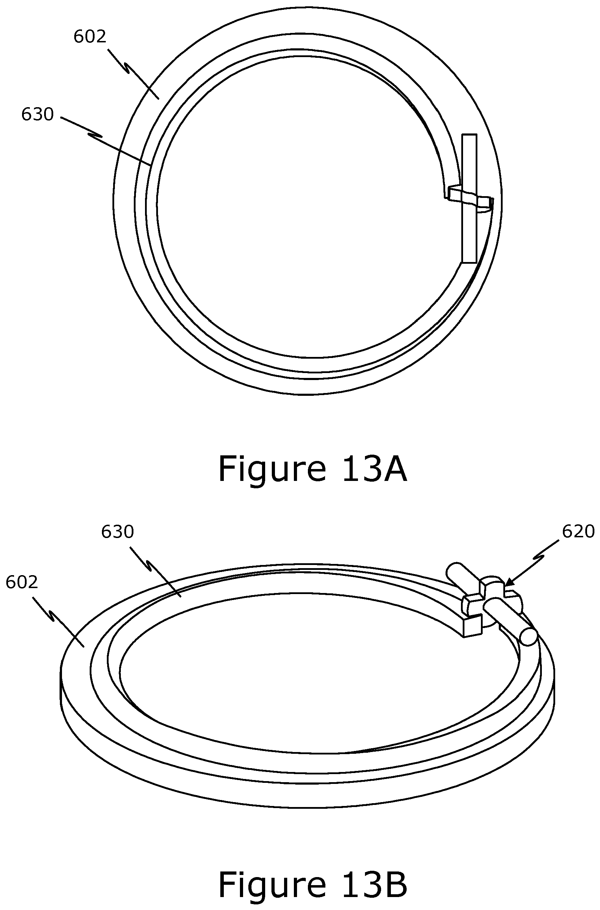

Figures 13A and 13B show a disc shaped body 602 and worm 620 in accordance with a sixth embodiment of the invention. The disc shaped body 602 has a single engagement feature 630 that extends the full circumference of the disc shaped body 602. The engagement feature 630 follows a spiral profile. The twist of the engagement features 630 maintains a snug engagement between two of the teeth of the worm 620. The disc shaped body 602 can be used with the counter ring 504 shown in Figures 10 and 11, in order to drive the counter ring 504 in a different plane.

## Claims

1. **A dose counter** (100) for registering a count of doses dispensed from an inhaler, comprising:
a counter ring (104) comprising a circumferential drive feature (106);
a worm (120) comprising a shaft (122) and a gear (124) extending in a helical path about the axis of the shaft (122) for driving the counter ring (104), wherein the gear (124) comprises one or more recesses distributed along the helical path;
a cylindrical body (102) comprising an engagement feature (130a, 130b) for driving the gear (124), the engagement feature extending in a helical or spiral path from the cylindrical body (102) to engage the gear (124) such that relative rotational movement between the cylindrical body (102) and the worm (120) rotates the worm about its shaft (122).

2. A dose counter as claimed in claim 1, in which the engagement feature has, orthogonal to its helical or spiral path, a cross section comprising an engagement shape, the engagement shape being configured to fit into each recess of the gear of the worm, wherein the orientation of the engagement shape changes along the path of the engagement feature maintaining the fit into at least one of the recesses of the gear as the worm rotates about its shaft.

3. A dose counter as claimed in claim 2, in which the engagement shape is configured to fit snugly into each recess.

4. A dose counter as claimed in any one of the preceding claims, in which the cylindrical body comprises a plurality of engagement features, the engagement features extending in rotationally parallel helical or spiral paths.

5. A dose counter as claimed in any one of the preceding claims, comprising a housing, the worm mounted in the housing.

6. A dose counter as claimed in any one of claims 1 to 5, wherein a feature of the counter ring is arranged to release a biasing component configured to engage with the cylindrical body to prevent further movement of the cylindrical body.

7. A dose counter as claimed in any one of claims 1 to 5, wherein the dose counter comprises a sprung metal component and a portion of the circumference of the counter ring comprises a gap for receiving the sprung metal component, wherein the sprung metal component is arranged to move into the gap when aligned with the gap, and into the path of the engagement feature to prevent further movement of the cylindrical body.

8. A dose counter as claimed in any of claims 1 to 5, comprising a spring and rigid locking component, wherein the spring is configured to engage with the engagement feature to prevent further movement of the cylindrical body, and the locking component is arranged to prevent disengagement of the of the spring once it has engaged with the engagement feature.

9. A dose counter as claimed in any of claims 1 to 5, wherein the counter ring comprises a locking clip, wherein the locking clip is arranged to be moveable by the worm to a position in which it is engaged with the engagement feature to prevent further movement of the cylindrical body.

10. A dose counter as claimed in any one of the preceding claims, wherein the counter ring and the cylindrical body have a common axis.

11. A dose counter as claimed in any one of the preceding claims, wherein the counter ring circumferential drive feature comprises regularly spaced teeth or ribs.

12. A dose counter as claimed in claim 11, wherein the circumferential drive feature extends part way around the circumference, and is interrupted by a discontinuity in the regular spacing of the teeth or ribs in the form of a wider gap or a filled in gap.

13. A dose counter as claimed in any one of the preceding claims, wherein the dose counter comprises a ratchet and the ratchet comprises a pawl configured to engage one of the counter ring, the worm or the cylindrical body to prevent back rotation of the counter ring.

14. **An inhaler** comprising a dose counter according to any preceding claim.

15. The inhaler of claim 14 wherein the inhaler is a soft mist inhaler (SMI) or a dry powder inhaler (DPI).

## Patentansprüche

1. **Dosiszähler** (100) zum Registrieren einer Anzahl von Dosen, die von einem Inhalator ausgegeben werden, umfassend:
einen Zählerring (104), der ein Umfangsantriebsmerkmal (106) umfasst;
eine Schnecke (120), umfassend eine Welle (122) und ein Zahnrad (124), das sich in einem schraubenförmigen Pfad um die Achse der Welle (122) zum Antreiben des Zählerrings (104) erstreckt, wobei das Zahnrad (124) eine oder mehrere Aussparungen, die entlang des schraubenförmigen Pfads verteilt sind, umfasst;
einen zylindrischen Körper (102), umfassend ein Eingriffsmerkmal (130a, 130b) zum Antreiben des Zahnrads (124), wobei das Eingriffsmerkmal sich in einem schraubenförmigen oder spiralförmigen Pfad von dem zylindrischen Körper (102) erstreckt, um das Zahnrad (124) derart in Eingriff zu nehmen, dass eine relative Drehbewegung zwischen dem zylindrischen Körper (102) und der Schnecke (120) die Schnecke um ihre Welle (122) dreht.

2. Dosiszähler nach Anspruch 1, wobei das Eingriffsmerkmal orthogonal zu seinem schraubenförmigen oder spiralförmigen Pfad einen Querschnitt aufweist, der eine Eingriffsform aufweist, wobei die Eingriffsform so konfiguriert ist, dass sie in jede Aussparung des Zahnrads der Schnecke passt, wobei sich die Ausrichtung der Eingriffsform entlang des Pfades des Eingriffsmerkmals ändert, wobei das Passen in mindestens eine der Aussparungen des Zahnrads aufrechterhalten, wenn sich die Schnecke um ihre Welle dreht.

3. Dosiszähler nach Anspruch 2, wobei die Eingriffsform so konfiguriert ist, dass sie in jede Aussparung passt.

4. Dosiszähler nach einem der vorhergehenden Ansprüche, wobei der zylindrische Körper eine Mehrzahl von Eingriffsmerkmalen aufweist, wobei sich die Eingriffsmerkmale in drehparallelen schraubenförmigen oder spiralförmigen Pfaden erstrecken.

5. Dosiszähler nach einem der vorhergehenden Ansprüche, umfassend ein Gehäuse, wobei die Schnecke in dem Gehäuse angebracht ist.

6. Dosiszähler nach einem der Ansprüche 1 bis 5, wobei ein Merkmal des Zählerrings so angeordnet ist, dass es eine Vorspannkomponente freigibt, die so konfiguriert ist, dass sie mit dem zylindrischen Körper in Eingriff kommt, um eine weitere Bewegung des zylindrischen Körpers zu verhindern.

7. Dosiszähler nach einem der Ansprüche 1 bis 5, wobei der Dosiszähler eine federnde Metallkomponente umfasst und ein Abschnitt des Umfangs des Zählerrings einen Spalt zum Aufnehmen der federnden Metallkomponente umfasst, wobei die federnde Metallkomponente so angeordnet ist, dass sie sich in den Spalt bewegt, wenn sie mit dem Spalt ausgerichtet ist, und in den Pfad des Eingriffsmerkmals, um eine weitere Bewegung des zylindrischen Körpers zu verhindern.

8. Dosiszähler nach einem der Ansprüche 1 bis 5, umfassend eine Feder und eine starre Verriegelungskomponente, wobei die Feder so konfiguriert ist, dass sie mit dem Eingriffsmerkmal in Eingriff kommt, um eine weitere Bewegung des zylindrischen Körpers zu verhindern, und die Verriegelungskomponente so angeordnet ist, dass sie ein Lösen der der Feder verhindert, sobald sie mit dem Eingriffsmerkmal in Eingriff getreten ist.

9. Dosiszähler nach einem der Ansprüche 1 bis 5, wobei der Zählerring eine Verriegelungsklammer aufweist, wobei die Verriegelungsklammer so angeordnet ist, dass sie durch die Schnecke in eine Position bewegbar ist, in der sie mit dem Eingriffsmerkmal in Eingriff steht, um eine weitere Bewegung des zylindrischen Körpers zu verhindern.

10. Dosiszähler nach einem der vorhergehenden Ansprüche, wobei der Zählerring und der zylindrische Körper eine gemeinsame Achse aufweisen.

11. Dosiszähler nach einem der vorhergehenden Ansprüche, wobei das Umfangsantriebsmerkmal des Zählrings regelmäßig beabstandete Zähne oder Rippen aufweist.

12. Dosiszähler nach Anspruch 11, wobei sich das Umfangsantriebsmerkmal teilweise um den Umfang erstreckt und durch eine Diskontinuität in der regelmäßigen Beabstandung der Zähne oder Rippen in Form eines breiteren Spaltes oder eines gefüllten Spaltes unterbrochen ist.

13. Dosiszähler nach einem der vorhergehenden Ansprüche, wobei der Dosiszähler eine Ratsche umfasst und die Ratsche eine Klinke umfasst, die so konfiguriert ist, dass sie mit einem von dem Zählerring, der Schnecke oder dem zylindrischen Körper in Eingriff kommt, um eine Rückdrehung des Zählerrings zu verhindern.

14. **Inhalator,** umfassend einen Dosiszähler nach einem der vorhergehenden Ansprüche.

15. Inhalator nach Anspruch 14, wobei der Inhalator ein Weichnebelinhalator, SMI, oder ein Trockenpulverinhalator, DPI, ist.

## Revendications

1. **Compteur de doses** (100) pour enregistrer un décompte de doses distribuées à partir d'un inhalateur, comprenant :
une bague de comptage (104) comprenant une caractéristique d'entraînement circonférentiel (106) ;
une vis sans fin (120) comprenant un arbre (122) et un engrenage (124) s'étendant dans un trajet hélicoïdal autour de l'axe de l'arbre (122) pour entraîner la bague de comptage (104), dans lequel l'engrenage (124) comprend un ou plusieurs évidements distribués le long du trajet hélicoïdal ;
un corps cylindrique (102) comprenant une caractéristique de mise en prise (130a, 130b) pour entraîner l'engrenage (124), la caractéristique de mise en prise s'étendant dans un trajet hélicoïdal ou en spirale à partir du corps cylindrique (102) pour mettre en prise l'engrenage (124) de sorte qu'un mouvement de rotation relatif entre le corps cylindrique (102) et la vis sans fin (120) fait tourner la vis sans fin autour de son arbre (122).

2. Compteur de doses selon la revendication 1, dans lequel la caractéristique de mise en prise a, orthogonalement à son trajet hélicoïdal ou en spirale, une coupe transversale comprenant une forme de mise en prise, la forme de mise en prise étant configurée pour s'ajuster dans chaque évidement de l'engrenage de la vis sans fin, dans lequel l'orientation de la forme de mise en prise change le long du trajet de la caractéristique de mise en prise en maintenant l'ajustement dans au moins l'un des évidements de l'engrenage à mesure que la vis sans fin tourne autour de son arbre.

3. Compteur de doses selon la revendication 2, dans lequel la forme de mise en prise est configurée pour s'ajuster étroitement dans chaque évidement.

4. Compteur de doses selon l'une quelconque des revendications précédentes, dans lequel le corps cylindrique comprend une pluralité de caractéristiques de mise en prise, les caractéristiques de mise en prise s'étendant dans des trajets hélicoïdaux ou en spirale parallèles en rotation.

5. Compteur de doses selon l'une quelconque des revendications précédentes, comprenant un logement, la vis sans fin étant montée dans le logement.

6. Compteur de doses selon l'une quelconque des revendications 1 à 5, dans lequel une caractéristique de la bague de comptage est agencée pour libérer un composant de sollicitation configuré pour venir en prise avec le corps cylindrique pour empêcher un mouvement supplémentaire du corps cylindrique.

7. Compteur de doses selon l'une quelconque des revendications 1 à 5, dans lequel le compteur de doses comprend un composant métallique à ressort et une portion de la circonférence de la bague de comptage comprend un espace destiné à recevoir le composant métallique à ressort, dans lequel le composant métallique à ressort est agencé pour se déplacer dans l'espace lorsqu'il est aligné avec l'espace, et dans le trajet de la caractéristique de mise en prise pour empêcher un mouvement supplémentaire du corps cylindrique.

8. Compteur de doses selon l'une quelconque des revendications 1 à 5, comprenant un ressort et un composant de verrouillage rigide, dans lequel le ressort est configuré pour venir en prise avec la caractéristique de mise en prise afin d'empêcher un mouvement supplémentaire du corps cylindrique, et le composant de verrouillage est agencé pour empêcher le dégagement de la prise du du ressort une fois qu'il s'est mis en prise avec la caractéristique de mise en prise.

9. Compteur de doses selon l'une quelconque des revendications 1 à 5, dans lequel la bague de comptage comprend une pince de verrouillage, dans lequel la pince de verrouillage est agencée pour pouvoir être déplacée par la vis sans fin vers une position dans laquelle elle vient en prise avec la caractéristique de mise en prise pour empêcher un mouvement supplémentaire du corps cylindrique.

10. Compteur de doses selon l'une quelconque des revendications précédentes, dans lequel la bague de comptage et le corps cylindrique ont un axe commun.

11. Compteur de doses selon l'une quelconque des revendications précédentes, dans lequel la caractéristique d'entraînement circonférentiel de la bague de comptage comprend des dents ou des nervures régulièrement espacées.

12. Compteur de doses selon la revendication 11, dans lequel la caractéristique d'entraînement circonférentielle s'étend à mi-course autour de la circonférence, et est interrompue par une discontinuité dans l'espacement régulier des dents ou des nervures sous la forme d'un espace plus large ou d'un espace rempli.

13. Compteur de doses selon l'une quelconque des revendications précédentes, dans lequel le compteur de doses comprend un rochet et le rochet comprend un cliquet configuré pour venir en prise avec l'un parmi la bague de compteur, la vis sans fin ou le corps cylindrique pour empêcher la rotation en arrière de la bague de compteur.

14. **Inhalateur** comprenant un compteur de doses selon l'une quelconque des revendications précédentes.

15. Inhalateur selon la revendication 14, dans lequel l'inhalateur est un inhalateur de brume ultra fine (soft mist inhaler, SMI) ou un inhalateur de poudre sèche (dry powder inhaler, DPI) .
